# EUROPÄISCHE PATENTSCHRIFT

(11) **EP 2 665 687 B1**
(45) Veröffentlichungstag und Bekanntmachung des Hinweises auf die Patenterteilung: **03.07.2019**
(21) Anmeldenummer: 11819045.3
(22) Anmeldetag: 21.12.2011
(51) Int. Cl.: C03B 1/02, C03C 1/02

(54) **VORBEHANDLUNG VON ROHMATERIAL ZUR HERSTELLUNG VON BASALTFASERN**
PRE-TREATMENT OF RAW MATERIAL FOR PRODUCING BASALT FIBERS
PRÉTRAITEMENT DE MATÉRIAU BRUT POUR LA FABRICATION DE FIBRES DE BASALTE

(30) Priorität: 22.12.2010 AT 21192010
(43) Veröffentlichungstag der Anmeldung: 27.11.2013
(73) Patentinhaber: ASA.TEC GmbH, A-3550 Langenlois (AT)
(72) Erfinder: SCHINKINGER, Thomas, A-4072 Alkoven (AT); MAYER, Anton, A-8700 Leoben (AT)
(74) Vertreter: Burger, Hannes
(86) Internationale Anmeldenummer: PCT/AT2011/050052
(87) Internationale Veröffentlichungsnummer: WO 2012/083335

(56) Entgegenhaltungen:
- WO-A1-00/73233
- WO-A1-01/98220
- WO-A1-2007/112471
- DD-A1- 200 567
- DE-A1- 2 536 122
- DE-A1-102005 040 268
- DE-T2- 69 607 206
- US-A- 3 941 574
- US-A- 5 472 917
- SPARIDAENS A J C M: "BATCH PELLETISATION-THE KEY TO GLASS QUALITY IMPROVEMENT*", GLASS TECHNOLOGY, SOCIETY OF GLASS TECHNOLOGY, SHEFFIELD, GB, Bd. 32, Nr. 5, 1. Oktober 1991 (1991-10-01), Seiten 149-152, XP000237923, ISSN: 0017-1050

## Beschreibung

Die Erfindung betrifft die Verwendung von artifiziell hergestellten Formkörpern hergestellt gemäß einem Verfahren zur Vorbehandlung von Ausgangsmaterial enthaltend Basalt und zumindest ein Bindemittel zur Herstellung einer mineralischen Schmelze für die Produktion von endlosen Mineralfasern zur Herstellung von endlosen Mineralfasern.

Als Mineralfasern werden üblicherweise erstarrte silikatische Fasern bezeichnet. Man unterscheidet im weitesten Sinne Kurzfasern, wie Stapelfasern, Steinwolle, etc. und endlose Fasern.

Zur Herstellung von endlosen Mineralfasern, wobei die am meist verwendeten Mineralfasern Glasfasern sind, werden üblicherweise Rohstoffe verwendet und unter hohen Energieaufwand geschmolzen. Glasschmelzen unterscheiden sich von Gesteinsschmelzen erheblich. Üblicherweise werden für die Herstellung endloser Fasern die Gesteinsrohstoffe unter Zugabe weiterer Komponenten geschmolzen um die Eigenschaften der Schmelze in gewünschter Weise zu verändern und an die jeweilige Verarbeitungs- und Formgebungstechnologie anzupassen. So beschreibt unter anderem die DE 195 38 599 B4 ein Verfahren zur Herstellung von Mineralfasern aus Gestein, glasartigen Industrieabfällen und technischen Glasabfällen, worin nach der mechanischen Trennung von nicht glashaltigen und überwiegend glashaltigen Produkten die überwiegend glashaltigen Produkte mit einer Teilchengröße von weniger als 80 mm in einer Schmelzwanne aufgeschmolzen werden, wobei die Schmelzwanne mit einem Speiserkanal in Verbindung steht.

Die DE 103 52 323 A1 beschreibt die Verwendung von Formsteinen gemeinsam mit Ergussgesteinen, wie Basalt und/oder Diabas zur Herstellung mineralischer Schmelze. Es ist ein Verfahren zur Herstellung einer mineralischen Schmelze für die Produktion von Mineralfasern, insbesondere von Steinwolle zur Wärme- und/oder Schalldämmung sowie für den Brandschutz, von Substraten für die Pflanzenzucht, von Verstärkungsfasern und von Fasern für Filtrationszwecke, bei dem zumindest aus der Produktion stammende Reststoffe sowie Korrekturstoffe zur Einstellung der erforderlichen Zusammensetzung und Viskosität der Schmelze zerkleinert und mit einem Bindemittel zu Formsteinen gepresst und die Formsteine einem Schmelzaggregat zugeführt werden beschrieben. In diesem Schmelzprozess werden geeignete Rohstoffe geschmolzen und anschließend die derart entstandene Schmelze in einem Zerfaserungsaggregat zerfasert. Das Zerfasern der Schmelze erfolgt beispielsweise in einem so genannten Zieh-, Schleuder- oder Blasverfahren. Steinwolle wird hauptsächlich aus Gemengen aus gebrochenen Ergussgesteinen, wie beispielsweise Basalt oder Diabas, und geringen Mengen an Kalkstein, Dolomit und Magnesit als Ergänzungsstoffe, sowie aus gebrochenen Ergussgesteinen und grobstückigen Hochofenschlacken sowie gegebenenfalls zusätzlichen geringen Mengen an Kalkstein, Dolomit und Magnesit hergestellt. Diese Ergänzungsstoffe können jeweils für sich allein oder in unterschiedlichen Mischungen miteinander den Gemengen beigefügt werden. In zunehmendem Masse werden die gebrochenen Rohstoffe durch künstlich hergestellte Körper entsprechender Größe, Form und Festigkeit ersetzt, die aus verschiedenen Roh- und Reststoffen sowie geeigneten Bindemitteln zusammengesetzt werden. Diese Körper werden als Formsteine bezeichnet. Die Formsteine können feinkörnig gebrochene natürliche Gesteine enthalten. Als weitere Komponenten kommen produktionsbedingte Reststoffe hinzu, beispielsweise die beim Herstellungsprozess zwangsläufig entstehenden gröberen Bestandteilen, wie Schmelzperlen, die bei der regelmäßigen Entleerung der Schmelzöfen anfallende erstarrte Schmelze mitsamt den teilweise aufgeschmolzenen Gesteinsresten und Teilen der Ofenauskleidung aus feuerfesten Baustoffen sowie den Dämmstoffen oder Substraten, die bei der Besäumung einer endlos hergestellten Faserbahn anfallen. Sonstige produktionsbedingte Reststoffe sind Verschnittreste, fehlerhafte Produkte oder aufzuschmelzende gebrauchte Dämmstoffe oder Substrate. Die produktionsbedingten Reststoffe werden für die Herstellung von Formsteinen aufbereitet, d.h. zerkleinert, gemahlen und anschließend mit Korrekturstoffen gemischt. Mit Hilfe dieser Korrekturstoffe wird die erforderliche Zusammensetzung der Gemenge erreicht, die ein gleichmäßiges und rasches Aufschmelzen in dem Schmelzaggregat bewirkt. Gleichzeitig werden dadurch die Temperatur und die Viskosität der entstehenden Schmelze so weit beeinflusst, dass ein möglichst wirkungsvoller, gleichmäßig ablaufender Zerfaserungsprozess erreicht wird. Korrekturstoffe sind beispielsweise Schlacken aus der Stahlindustrie wie Konverter- oder Gießpfannenschlacken oder Schmelzkammergranulate aus Kohlekraftwerken. Als hier wesentliche Korrekturstoffe gelten auch Stoffe, die Aluminium in oxidischer und/oder in metallischer Form enthalten. Geeignete Trägerstoffe sind einmal Roh-Bauxit oder calcinierter Bauxit, sowie Tonerdeschmelzzemente, die naturgemäß auch die Funktion eines Bindemittels erfüllen können. Die körnigen und faserigen Komponenten, die internen Reststoffe und die Korrekturstoffe werden überwiegend mit anorganischen Bindemitteln, zumeist unter Zusatz von Wasser, gemischt und anschließend zu Formkörpern verpresst. Nach Erreichen einer für die Lagerung im Haufwerk, der Förderung und Beschickung ausreichenden Festigkeit der Formsteine, im Allgemeinen sollen die Formsteine nach beispielsweise 3 Tagen eine Mindest-Druckfestigkeit von ca. 1-5 MPa erreichen, werden diese zusammen mit den anderen Rohstoffen oder allein, jedoch immer zusammen mit für den Schmelzvorgang erforderlichen stückigen Brennstoffen, dem Schmelzaggregat aufgegeben. In diesem wird die für die Faserbildung erforderliche Schmelze hergestellt, die sodann dem Zerfaserungsaggregat zugeführt wird. Bestandteile der Formsteine, insbesondere die Korrekturstoffe und/oder sonstige Gemengebestandteile können zumindest teilweise durch körnige Verbrennungsrückstände, insbesondere Aschen oder Schlacken, aus der Verbrennung von vorzugsweise Braunkohlen- und/oder Steinkohlenstäuben, Papierschlamm oder Holzspänen substituiert werden.

Die DE 25 36 122 C2 beschreibt ein Verfahren zum Einsatz von Basalt in Schmelzöfen zur Herstellung von Mineralwolle durch Feinzerkleinern von Basalt auf weniger als 3 mm große Stücke und Zusatz eines Bindemittels und Verpressen zu Briketts. Dadurch sollen die natürlichen Schwankungen des Rohbasalts ausgeglichen werden, dass der störende Einfluss von groben Einschlüssen und Akkumulationen im Basalt und von Dolomitakkumulationen durch das dem Brikettieren vorangehende weitgehendes Zerkleinern gemindert wird.

Die WO 01/98220 A1 beschreibt Briketts zur Herstellung von Mineralwolle. Die Zusammensetzung der Briketts variiert abhängig von der vorgesehenen Verwendung. Die Briketts werden durch Mischen von Mineralmaterial mit einem Bindemittel und falls erforderlich durch Zugabe von Wasser hergestellt. Die Briketts können durch Pressen hergestellt werden. Der Aushärteprozess kann durch Erhitzen beschleunigt werden.

In DD 2005 675 wird ein Verfahren zur Herstellung anorganischer Fasern für Dämmstoffe aus Roh- und Industrieabfallstoffen beschrieben, wobei als Rohstoff Tonschiefer mit weiteren Roh- und/oder Industrieabfallstoffen, wie Basalt, Dolomit, Diabas, Hüttenschlacken zerkleinert und gemischt werden, worauf das Gemisch gegebenenfalls nach vorangegangener Formgebung in Schmelzeinrichtungen geschmolzen und zerfasert wird. Die Mischungsbestandteile werden feinkörnig oder staubförmig eingesetzt und mittels Granulier- oder Brikettiertechnik stückig gemacht. Die Formlinge werden vor dem Einsatz in der Schmelzeinrichtung einer Trocknung, Vorerhitzung und/oder Verfestigung unterworfen. Trotz der erhöhten Viskosität gegenüber einer reinen Basaltschmelze können Fasern mit verringertem Durchmesser, größerer Schlankheit, höherer Rekristallisationstemperatur und vermindertem Schmelzperlengehalt gewonnen werden. Durch die Verwendung von ausgeblähtem Schiefermaterial lassen sich herkömmliche Fasertechnologien verwenden. Durch den Einsatz von Tonschiefer wird über die bewirkte Spreizung des Plastizitätsintervalles zwischen Erweichungs- und Schmelztemperatur eines Tonschiefers enthaltenden Gemisches gegenüber gebräuchlichen Einsatzstoffen eine Verbesserung der Faserqualität verzeichnet.

In der US 3,941,574 A wird die Bereitstellung und hydrothermische Behandlung einer Ausgangsmenge von Basalt, Diatomit, Calcium und Magnesiumhydroxid zur Herstellung von Glasfasern beschrieben. Die Bestandteile werden in einem wässrigen Medium vermischt und 20 Minuten einer Temperatur von 50°C bis 200°C oder 30 Minuten 100°C ausgesetzt. Der resultierende Brei wird getrocknet und granuliert. Das resultierende Gut wird geschmolzen. Die Fasern weisen eine höhere Stabilität beim Filament ziehen auf.

Die DE 10 2005 040 268 A1 betrifft Formkörper für die Erzeugung zu zerfasernder mineralischer Schmelze zur Herstellung von Dämmstoffen, insbesondere Steinwolle. Die Formkörper können einen Überzug voll- oder teilflächig aufweisen. Die Ummantelung kann aus einer Gesteinsfraktion bestehen, die mit hydraulischen Bindemitteln gebunden ist.

Die WO 2007/112471 A1 beschreibt ein Herstellungsverfahren für endlose Mineralfasern aus Rohmaterialien wie etwa Gestein, insbesondere Basaltgestein, Gemengen auf dessen Basis, glashaltigen industriellen und technischen Abfallprodukten, bei dem die genannten Materialien in einem Schmelzofen aufgeschmolzen werden und die Schmelze Fließspeisern in der Verarbeitungszone zugeführt werden, wobei in der Verarbeitungszone ein Entnahmebereich für die Schmelze zur Fließzufuhr an die Düsen angeordnet ist. Die Qualität der Endlosfaser und der Ausstoß bei ihrer Verarbeitung hängen von der Homogenität der den Düsen zugeführten Schmelze ab. Es ist daher zweckdienlich, dies in einer ausgewählten Zone anzustreben, der Entnahmebereich genannt wird.

Aufgabe der vorliegenden Erfindung ist es ein Verfahren für die Produktion von endlosen Mineralfasern zur Verfügung zu stellen, mit welchem eine hohe Produktivität erzielt werden kann.

Die Aufgabe der Erfindung wird durch die Verwendung von artifiziell hergestellten Formkörpern hergestellt gemäß einem Verfahren zur Vorbehandlung von Ausgangsmaterial zur Herstellung einer mineralischen Schmelze für die Produktion von endlosen Mineralfasern, insbesondere Basaltfasern,wobei das Ausgangsmaterial, welches Basalt und zumindest ein Bindemittel, ausgewählt aus einer Gruppe umfassend Wasser, Ton, Bentonit, Sulfitlauge, Zellulosederivate, Saccharide, Zucker, Stärke, Melasse, Zement, Phosphate, siliziumorganische Substanzen, in einer Menge mit einer unteren Grenze von 1 % und einer oberen Grenze von 40 %, Quarzsand und Schlacke, insbesondere Hochofenschlacke umfasst, zu Partikeln gemahlen wird, aus den Partikeln Formkörper hergestellt werden und die Formkörper bei einer Temperatur von ca. 300°C+50°C getempert werden, gelöst. Vorteilhaft dabei erweist sich, dass gleichmäßige Bedingungen in der Gesteinsschmelze geschaffen werden können im Vergleich zur herkömmlichen Schmelzverfahren, wo die Rohstoffe in unkompaktierter Form in den Schmelzofen eingebracht werden, und somit die unterschiedlichen Schmelztemperaturen der unterschiedlichen Rohstoffe zum Tragen kommen. Zudem wird eine Verstaubung bzw. ein Ausblasen von feinkörnigen Zusätzen, beispielsweise durch Flammengase, und/oder Entmischung unterschiedlicher Ausgangsmaterialien bzw. Rohstoffe im Schmelzofen verhindert. Auch die MAK-Werte können dadurch leichter eingehalten werden. Durch die homogenen Eigenschaften der in den Schmelzofen eingebrachten Formkörper lassen sie gleichmäßige Eigenschaften der Schmelze erzielen und somit auch gleichmäßige endlose Mineralfaser herstellen, die wiederum die Gebrauchseigenschaften der Mineralfasern und der daraus hergestellten Mineralfaserprodukte günstig beeinflussen.

Durch die Kompaktierung zu Formkörper gelangen die einzelnen Partikel so knapp zueinander, dass sie schneller reagieren können im Vergleich zu losen Partikeln, wo dazwischen Hohlräume vorliegen.

Durch die Verwendung der erfindungsgemäß hergestellten Formkörper zur Herstellung endloser Mineralfasern, insbesondere Basaltfasern, ergibt sich auch eine bessere Dosierbarkeit. Zudem findet bereits eine Vorreaktion vor dem vollständigen Aufschmelzen der Ausgangsmaterialien im Schmelzofen statt, woraus höhere Festigkeiten als bei üblichen Glas- und Basaltfaserwerkstoffen resultieren.

Von Vorteil erweist sich, dass das Ausgangsmaterial eine Restfeuchte mit einer unteren Grenze von 1 % und einer oberen Grenze von 20 % aufweist, wodurch ein Verkleben der Mühle beim Mahlen verhindert bzw. verzögert werden kann.

Vorzugsweise wird das Ausgangsmaterial zu Partikeln mit einer Größe von weniger als 500 µm, insbesondere 200 µm, vorzugsweise 100 µm, gemahlen, wodurch die Herstellung einheitlicher Formkörper erleichtert wird.

Die Formkörper können durch Granulierung bzw. Pelletierung, Extrusion, Sprühtrocknung, Expandierung, etc. hergestellt werden, wodurch eine bessere Verarbeitbarkeit des Ausgangsmaterials ermöglicht wird.

Die Herstellung der Formkörper kann durch Zugabe eines Bindemittels ausgewählt aus einer Gruppe umfassend Wasser, Ton, Bentonit, Sulfitlauge, Zellulosederivate, Saccharide, Zucker, Stärke Melasse, Phosphate, Zement, siliziumorganische Substanzen in einer Menge mit einer unteren Grenze von 1 % und einer oberen Grenze von 40 % durchgeführt werden, wodurch auch feinkörnige Zusätze in das Gemenge eingebracht werden können ohne dass diese im weiteren Herstellungsprozess verstauben bzw. ausgeblasen werden.

Die Formkörper können zumindest bereichsweise mit einer Beschichtung mit Stoffen ausgewählt aus einer Gruppe umfassend Mineralmehl, Filterstaub, Flugasche, Schlackenmehl, Ton versehen werden, wodurch zumindest teilweise gerundete und beschichtete Formkörper entstehen, die vorteilhaft in den Schmelzofen eingesetzt werden können.

In einer Weiterbildung der Erfindung kann eine Bindung der Beschichtung durch das und/oder andere Bindemittel, wie organische Bindemittel ausgewählt aus einer Gruppe umfassend Stoffe auf Basis von Zucker, Lignin, Ligninsulfonate und/oder anorganische Bindemittel ausgewählt aus einer Gruppe umfassend Stoffe auf Basis von Zement, Kieselsäurelösungen durchgeführt werden, wodurch ein verbessertes Anhaften der Beschichtung auf den Formkörpern erzielt werden kann.

Ferner kann zumindest ein Beschleuniger in einer Menge mit einer oberen Grenze von 20 % und einer unteren Grenze von 0,1 % zur Herstellung und/oder Beschichtung der Formkörper beigemengt werden, wodurch der Herstellungs- bzw. Beschichtungsprozess verkürzt werden können. Zudem können durch die Beschleunigersubstanz die chemischen bzw. physikalischen Eigenschaften der Formkörper verbessert werden. Als Beschleuniger können beispielsweise jene der Zementindustrie verwendet werden.

Besonders vorteilhaft erweisen sich Formkörper, insbesondere Grüngranulat, mit einer Größe mit einer oberen Grenze von 30 mm und einer unteren Grenze von 1 mm, vorzugsweise 10 mm, weil dadurch der Energieeintrag zum Aufschmelzen der Formkörper im Schmelzofen gering gehalten werden kann ohne gleichzeitig die Schmelzeigenschaften negativ zu beeinflussen.

Die Formkörper werden bei einer Temperatur mit einer oberen Grenze von 1000°C und einer unteren Grenze von 50°C getempert, wodurch einerseits bessere physikalische Eigenschaften, wie höhere Abriebfestigkeit und geringere Abplatzungen erzielt werden können und andererseits der Platzbedarf für die Lagerung zur Trocknung entfällt.

Einführend sei festgehalten, dass in den unterschiedlich beschriebenen Ausführungsbeispielen gleiche Teile mit gleichen Bezeichnungen versehen werden, wobei die in der gesamten Beschreibung enthaltenen Offenbarungen sinngemäß auf gleiche Teile mit gleichen Bezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Unter Basalt wird im Zusammenhang mit der Erfindung ein basisches Ergussgestein verstanden. Es besteht vor allem aus einer Mischung von Eisen-und Magnesium-Silikaten mit Olivin und Pyroxen sowie calciumreichem Feldspat (Plagioklas). Vorherrschende Mineralgruppen sind Plagioklase, Pyroxene, wie überwiegend als Augit oder Diopsid. Olivine, Biotit, Amphibolite, wie Hornblende, Alkalifeldspat, Quarz, Hornfels, und Foide treten in einigen Basaltvorkommen auf. Auch Diabas, Melaphyr, Foidit, Latit, Phonolith, Melilithit, Pikrit, Tephrit, Andesit, Porphyr, Spilit, Trachyt, etc. werden im Zusammenhang mit der Erfindung unter Basalt verstanden. Insgesamt erweisen sich Basalte von ihrer chemischen Zusammensetzung her als ausgesprochen variable Gesteine, was im Widerspruch zu ihrem recht einheitlichen Aussehen steht.

Im Zusammenhang mit der Erfindung handelt es sich bei den Mineralfasern, insbesondere Endlosmineralfasern, um MMMF (man made mineral fibers) - also synthetisch hergestellte Mineralfasern.

Das Ausgangsmaterial kann neben Basalt und zumindest einem Bindemittel auch Quarzsand, Ton, Kaolin, Hochofenschlacke, etc. umfassen und wird in einem ersten Arbeitsschritt gemahlen, wobei es allerdings bereits vor dem Mahlen gemischt werden kann. Als Ausgangsmaterial können auch Minerale und Rohstoffe ausgewählt aus einer Gruppe umfassend Metallverbindungen, wie Mangan-, Titan-, Kupfer-, Cobaltverbindungen, Alkaliverbindungen, Natrium-, Kaliumverbindungen Erdalkaliverbindungen, wie Magnesium- bzw. Bariumverbindungen. eingesetzt werden.

Selbstverständlich kann das Ausgangsmaterial auch erst während des oder nach dem Mahlen gemischt werden. Das Misch- und Mahlgut des Ausgangsmaterials kann auf ca. 50 % Basalt, ca. 20 % Hochofenschlacke, ca. 30 % Quarzsand und Ton basieren. In alternativen Ausführungsvarianten kann das Ausgangsmaterial auch nur Basalt, bzw. nur ausgewählte Zusatzstoffe wie nur Hochofenschlacke, Quarzsand oder Ton umfassen. Es ist auch möglich weitere Stoffe dem Ausgangsmaterial beizumengen. Nähere Ausführungen zur Zusammensetzung des Ausgangsmaterial können der Patentanmeldung "Rohmaterial zur Herstellung von Basaltfasern" der Anmelderin vom 23.12.2010 entnommen werden.

Aus dem Mahlgut werden Formkörper hergestellt, wobei ein Formkörper ein Aggregat aus zumindest 3 Partikel ist.

Die Mahlung kann beispielsweise in einer Durchlaufmühle DM1230 im geschlossenen Sicht-Kreislauf trocken durchgeführt werden, wobei der Durchmesser der Mühle 1,2 m und die Länge 3,0 m beträgt sowie 12 Hubleisten angeordnet sind und von ca. 5 t kugeln als Mahlkörper gemahlen wird. Die gemahlenen Partikel werden in einer Austragswand mit Schlitzen gewonnen. Zudem kann ein Sichter angeordnet sein. Die Partikelgrößenverteilung zeigt dass annähernd 80 % der Partikel eine Größe von 20 µm bis 100 µm aufweisen. Das Ausgangsmaterial wird vorzugsweise aus Silos oder Big-Bag-Stationen abgezogen. Ferner kann es mittels Dosierbandwaage und Schnecke auf die Mühle gegeben werden. Nachdem es die Mühle durchlaufen hat, gelangt es über die Austragswand, Schnecken und dem Becherwerk zur Aufgabeschnecke des Sichters. Dort wird es auf den Sichter aufgebracht und die Partikel mit der gewünschten Zielfeinheit in Big-Bag-Stationen gefördert. Das zu grobe Material (Griese) gelangt über Schnecken wieder in den Mahlraum und wird so lang gemahlen bis es die gewünschte Partikelgröße erreicht.

In einer alternativen Ausführungsvariante werden Partikel mit einer Größe von 200 µm zur Bildung der Formkörper verwendet, die durch eine offene Mühlenfahrweise hergestellt werden.

Es werden Formkörper mit unterschiedlichen Verfahren wie Granulieren, Pelletieren, Extrudieren, Expandieren, Sprühtrocknung, etc. hergestellt, die fest, förder- und dosierfähig sind. Die Formkörper weisen vorzugsweise eine kompaktierte Form auf.

Beim Granulieren werden entweder große Partikel oder sehr kleine Partikel mit unterschiedlicher Partikelgröße (Pulver) in ein Haufwerk mit Partikeln enger Partikelgröße verwandelt.

Vorteilhaft bei der Verwendung eines Granuliermischers erweist sich, dass die Herstellung der Ausgangsmasse für die Formkörper sowie die Granulierung und das Pudern in einer Apparatur, ohne zusätzlichen Handlingaufwand durchgeführt werden können. Die erzeugten Formkörper, insbesondere Granulat ist mechanisch vergleichsweise stabil. Der Eirich Intensivmischer R02, R05/T oder R11 eignet sich durch seine Konstruktion nicht nur zum einfachen Mischen von pulverförmigen Materialien bzw. Partikeln, sondern auch zu deren Granulierung. Von Vorteil ist, dass hiermit ein homogenes Kornband hergestellt werden kann. Der Mischer R11 besitzt ein Füllvolumen von 250 l und einen Wirbler-Drehzahlbereich von 80 rpm bis 800 rpm.

Dazu tragen maßgeblich der spezielle Mischrotor und der schräg gestellte mitdrehende Mischtopf des Mischers bei.

Der Mischer, als Hauptaggregat der Granulieranlage, wird in der Regel, abhängig vom Material, rezeptgenau mittels SPS Steuerung im Automatikbetrieb befüllt und betrieben. Dabei können mehrere verschiedene Ausgangsmaterialien, angeliefert im Big-Bag und/oder Silofahrzeug, verarbeit werden. In einem Ausführungsbeispiel wird das Ausgangsmaterial wird mittels pneumatischem Förderer von der Rezeptwaage in Vorlagebehälter transportiert und dann in den Mischer gefüllt. Voraussetzung für den Automatikbetrieb ist, neben einem geeigneten Granulierverhalten des zu verarbeitenden Ausgangsmaterials, die genaue Kenntnis der optimalen Granulierparameter, wie Mischablauf, Mischzeiten, Drehzahlen, Energieeintrag, etc.

Anstelle des Automatikbetriebs kann die Granulieranlage auch im Handbetrieb betrieben werden, wie z.B. bei komplizierten Granulierprozessen, dem Test neuer Prozesse sowie bei geringen Mengen, um das Granulierergebnis sicherstellen zu können, die optimalen Verfahrensparameter ermitteln zu können sowie um die Fixkosten für Kleinversuche (z.B. Vorbereitungs- und Reinigungsaufwand) zu minimieren.

Um die Granulierung anzuregen muss aber zudem ein Bindemittel, vorzugsweise Wasser beigefügt werden. Es wird eine definierte Menge mit einer unteren Grenze von 60 % und einer oberen Grenze von 99 % Trockenstoff, wie z.B. Ausgangsmaterial in Partikel- bzw. Mehlform, Basaltspartikel bzw. -mehl, mit einer bestimmten Menge mit einer unteren Grenze von 1 % und einer oberen Grenze von 40 % Wasser in den Mischtopf aufgegeben.

Nach dem Starten des Mischvorgangs wird der Trockenstoff mit dem Bindemittel vermischt und durch den kurzzeitig max. hochdrehenden Mischrotor in so genanntes Mikrogranulat zerschlagen.

Das Mikrogranulat ist zur Züchtung des Endproduktes nötig. Nach der Zerschlagungsphase (Mikrogranulatherstellung) wird die Drehzahl des Mischrotors verringert um ein Wachsen der Formkörper zu erwirken. Der Granuliervorgang wird bei Erstversuchen ständig durch Probennahme überwacht bis die gewünschte Granulatgröße erreicht ist. Sodann wird die Drehzahl des Mischrotors und des Mischtopfes für max. 30 Sekunden drastisch reduziert, in dieser Zeit muss falls nötig das zu feuchte Granulat durch Zugabe von Trockenstoff abgebunden werden.

Zu den Partikeln wird das Bindemittel, vorzugsweise Wasser, in einen Mischrotor beigegeben und nachfolgend der Mischrotor kurzzeitig mit einer sehr hohen Drehzahl betrieben, um Mikrogranulat herzustellen und anschließend der Mischrotor mit einer niedrigeren Drehzahl betrieben. Die Partikeln werden in einer Menge mit einer unteren Grenze von 70 % und einer oberen Grenze von 95 % und Bindemittel in einer Menge mit einer unteren Grenze von 5 % und einer oberen Grenze von 30 % zugegeben und für einen Zeitraum mit einer unteren Grenze von 10 Sekunden und einer oberen Grenze von 30 Minuten gemischt. Zu den Partikeln wird in den Mischrotor bei niedriger Drehzahl beispielsweise gemahlenes Ausgangsmaterial zugegeben, um das Grüngranulat abzubinden.

Der Granuliervorgang muss nach dem Abbinden (Abpudern) mit Trockenstoff schnellst möglich gestoppt werden, da sich unter Umständen größere Klumpen bilden können. Sollte dies geschehen, kann man diese bei einer geringen Anteilzahl von Hand aussortieren. Sind zu viele Klumpen vorhanden, ist es möglich durch erneutes durchführen des Mischprogramms die Charge zu retten.

Bei der Trockenstoffzugabe ist aber auch darauf zu achten, dass nicht zuviel zugegeben wird, da sonst der zu hohe Feinstoffanteil in den Nachfolgeschritten zu starker Staubentwicklung führt.

Nach Beendigung des Granulierprozesses kann das Granulat getrocknet bzw. getempert werden.

Für die Aufgabe über ein Förderband in einen Vorschmelzofen hat sich eine Granulatgröße von 5 mm bis 8 mm als vorteilhaft erwiesen.

Wichtig für eine annähernd gleich bleibende Granulatqualität ist die Vorraussetzung, dass der Trockenstoff immer trocken zu verarbeiten ist. Sollte der Trockenstoff zu feucht sein, wird der für die Rezeptur zuvor ermittelte Bindemittelanteil zu hoch sein. Hierdurch wird der Mischablauf gestört und es muss durch Korrektur, wie Handzugabe von Trockenstoff, das Gemenge nachreguliert werden.

Zur Bildung stabförmiger Formkörper kann beispielsweise ein Schwenkbalkengranulator verwendet werden, wobei die pastöse Gesteinspartikelmasse durch ein Lochblech gepresst wird. Ein solcher Schwenkbalkengranulator ist aus dem Stand der Technik bekannt ist (z.B. Fa. EL-A Schwenkbalkengranulator). Mit dem ELA Schwenkbalkengranulator können stabförmige Formkörper hergestellt werden. Je nach Extrusionsplatte (Lochplatte) variiert der Stabdurchmesser von 6 mm bis 15 mm. Die Länge des Stabgranulats kann nicht direkt beeinflusst werden, sondern wird durch die Brucheigenschaft der Extrusionsmasse (Basaltsmasse) bestimmt bzw. hervorgerufen.

Bevor mit der Granulation begonnen werden kann muss zuvor in einem extra Schritt die Granulatmasse hergestellt werden. Für Versuchsmengen kann die Masse von Hand in einer Mischwanne angerührt werden. Bei größeren Mengen wird dies von einem Mischer übernommen. Beim Anrühren der Masse ist darauf zu achten, dass diese die richtige Konsistenz besitzt. Ist die Masse zu weich bricht das Granulat nicht und es entstehen lange Stangen, die zudem miteinander verkleben. Wird das Granulat zu fest angerührt, ist es nicht mehr möglich die Masse durch die Lochplatte zu drücken. Genau hierin liegt eine Schwachstelle dieses Systems, um ein akzeptables Granulierungsergebnis zu erhalten, Um ein günstiges Verhältnis der Granulatlänge in Bezug zum Durchmesser herzustellen, muss die Granulatmasse zu einer festeren Konsistenz angerührt werden. Dies führt bei der Verarbeitung der Masse dazu, dass der Granulator nach einigen Extrusionshüben verstopft. Aus diesem Grund und dem des zusätzlich vorhergehenden Mischprozesses ist dieses System aufwändiger.

Bei der Extrusion werden zähflüssige härtbare Materialien in einem kontinuierlichen Verfahren durch eine speziell geformte Düse gepresst. Es entstehen Körper mit dem Querschnitt der Düse in beliebiger Länge.

Hierzu wird gemahlenes Ausgangsmaterial mit Bindemittel, insbesondere Wasser, versetzt um eine Ausgangsmasse herzustellen. Um eine gut verarbeitbare Ausgangsmasse zu erhalten, die nicht im Extruder klebt aber noch in den Apparat eingezogen wird, kann ein weiteres Fluid, vorzugsweise ein Tensid, zugegeben werden. Dieses wird dem Extruder zugeführt, wo die Extrusion erfolgt. Um transport- und dosierfähige Pellets zu erhalten wird das Extrudat vorzugsweise gepudert. Die bei der Extrusion hergestellten mehr oder weniger großen, stäbchenförmigen Pellets sind bei Trocknung bzw. beim Tempern mechanisch instabiler als Granulat und neigen daher eher zu Bruch und zur Bildung von Feinkorn.

Unter Expansion (Wärmeausdehnung) versteht man die Änderung der geometrischen Abmessungen (Länge, Flächeninhalt, Volumen) eines Körpers, hervorgerufen durch eine Veränderung seiner Temperatur. Die Umkehr dieses Vorganges durch die Abkühlung wird oft als Wärmeschrumpfung bezeichnet. Der Kennwert ist der Ausdehnungskoeffizient.

Die Sprühtrocknung (auch Zerstäubungstrocknung) ist eine Methode aus der Verfahrenstechnik zur Trocknung von Lösungen, Suspensionen oder pastösen Massen. Durch verschiedene Typen Düsen im Sprühkopf eines Sprühtrockners wird mittels einer Düse (durch Flüssigkeitsdruck, Pressluft oder Inertgas betrieben) oder rotierenden Zerstäuberscheiben (4.000-50.000 U/min) das zu trocknende Gut in einen Heißluftstrom (Temperaturen je nach Apparatur bis zu 220 °C) eingebracht, der es in Bruchteilen einer Sekunde zu einem feinen Pulver trocknet.

Beim Tempern, insbesondere einer Trocknung, der Formkörper, insbesondere des Grüngranulats, soll ein möglichst kontinuierliches Verfahren sowie eine hohe Trockenleistung erzielt werden. Zum Tempern der Formkörper kann ein Kammerofen, Fließbetttrockner, Bandtrockner oder dergleichen verwendet werden. Das Tempern erfolgt vorzugsweise bei einer Temperatur mit einer unteren Grenze von 50 °C und einer oberen Grenze von 1000°C für einen Zeitraum von 2 bis 15 Minuten, wobei eine Restfeuchte von ca. 1 % der Formkörper erzielt werden soll. Vorzugsweise betragen die Temperaturen ca. 300°C±50°C.

Als Trockner eignet sich beispielsweise der Kammerofen der Firma Hofmann Wärmetechnik und Ernst Reinhardt GmbH. Die zu trocknenden Formkörper werden dabei in einer Höhe von ca. 35 bis 40 mm auf Trockenbleche aufgegeben und dann für 3 Stunden bei 250°C im Ofen getrocknet. Nach Abkühlung der Formkörper dürfen diese eine Restfeuchte von max. 1% aufweisen. Es ist somit kein kontinuierlicher Prozess möglich und die Trockenleistung liegt bei weniger als 100 kg/h.

Als Bandtrockner kann beispielsweise ein EAL Versuchsbandtrockner BT3/2 verwendet werden. Er kann ein ELT Transportband, Runddrahtgeflecht, Drahtösengliederband der Fa. Märtens aufweisen, luft- und flüssigkeitsdurchlässig, temperaturbeständig sein. Es können Lufttrockenhauben, Infrarottrockenhauben, etc. angeordnet sein. Bandtrockner weisen eine hohe Trockenleistung auf, z.B. 170 kg/h oder 220 kg/h, bei einem Restfeuchtegehalt von unter 1 %. Zudem sind eine direkte Materialaufgabe und ein kontinuierlicher Betrieb bei gleichzeitig geringer Staubentwicklung und geringem Energieverbrauch (6kW) möglich. Die maximale Trocknungstemperatur liegt bei vorzugsweise 160°C bis 250°C.

Ein Fließbetttrockner hat eine hohe Trockenleistung von 200 kg/h und ermöglicht einen kontinuierlichen Betrieb durch direkte Materialaufgabe, wobei allerdings ein hoher Platzbedarf besteht. Der Energieverbrauch ist mit 140 kW sehr hoch und durch eine Vibrationsrinnenförderung reiben sich die Granulate gegenseitig ab, wodurch ein hoher Staubanteil entsteht.

Auch ein Warmlufttrockenband ist zur Trocknung des Grüngranulats bedingt geeignet, wobei die Trockenleistung sehr niedrig ist.

Das Tempern des Grüngranulats kann auch in einem Drehrohrofen, insbesondere einem direkt beheizten Drehrohrofen, erfolgen. Der Drehrohrofen kann direkt mit der Granulieranlage gekoppelt sein, beispielsweise über Förderbänder und Becherwerk das Grüngranulat in einen Pufferbehälter fördern, und ermöglicht eine kontinuierliche thermische Behandlung. Die zu trocknenden Formkörper können fein- bis grobkörnig oder stückig sein. Der direkt beheizte Drehrohrofen wird mit einem Erdgasbrenner befeuert. Die zu tempernden Formkörper werden mittels geeigneter Dosiereinrichtung, z.B. Dosierbandwaage, direkt, z.B. mittels Förderbänder, in den Ofen gefördert. Die am Ofenauslauf gewonnenen Formkörper fallen über einen Schacht zum nachfolgenden Drehrohrkühler und werden gekühlt und je nach Bedarf entsprechend weiterverarbeitet und verpackt.

Vorzugsweise erfolgt das Tempern des Grüngranulats im direkt beheizten Drehrohrofen bei einer Starttemperatur von 300°C (Temperatur in der Sintere, ca. 2,6 m vor Ofenauslauf) und einem Aufgabedurchsatz von 400 kg/h. Die angegebene Temperatur ist eine Mischtemperatur aus Gas- und Materialtemperatur, die einem gewissen Schwankungsbereich unterliegt. Das bei 300°C getrocknete Granulat ist fest, förder- und dosierfähig. Auch Starttemperaturen von 200°C und 400°C und Tempertemperaturen von 300°C sowie eine Durchsatz zwischen 300 kg/h und 500 kg/h ergibt ein zufrieden stellendes Ergebnis. Durch das Tempern im ausgemauerten, direkt beheizten Drehrohrofen wird keine signifikante Erhöhung des Feinkornanteils gegenüber dem ungetrockneten Granulat festgestellt.

In einer Weiterbildung der Erfindung können die Temperanlagen mit einer Klassierstation gekoppelt sein, womit eine kontinuierliche Produktklassierung möglich ist und Fehlformkörper für einen eventuellen Recyclingprozess ausgekreist werden können.

In einer Weiterbildung der Erfindung können Formkörperherstellungsanlagen und/oder die Temperanlagen mit einer weiteren Station gekoppelt sein, wo eine Beschichtung der Formkörper erfolgen kann. Die Beschichtung der Formkörper kann allerdings auch bereits in der Formkörperherstellungsanlage, wie Eirich Intensivmischer, Extruder, etc. oder der Temperanlage, wie Bandtrockner, Kammerofen, Drehrohrofen, etc. stattfinden. Die Beschichtung kann mit Stoffen ausgewählt aus einer Gruppe umfassend Mineralmehl, Filterstaub, Flugasche, Schlackenmehl, Ton, durchgeführt werden. Die Bindung der Beschichtung kann durch Bindemittel, wie organische Bindemittel ausgewählt aus einer Gruppe umfassend Stoffe auf Basis von Zucker, Melasse, Lignin, Ligninsulfonate und/oder anorganische Bindemittel ausgewählt aus einer Gruppe umfassend Wasser, Stoffe auf Basis von Zement, Bentonit, Sulfitlauge, Kieselsäureverbindungen erfolgen. Die so beschichteten und somit auch teilweise gerundeten Formkörper können vorteilhaft in den Schmelzofen eingesetzt werden.

Zudem können Ventilatoren angeordnet sein, die geeignet sind Staub und granulatartige Materialien abzusaugen. Vorzugsweise werden Ventilatoren mit einem hohen Wirkungsgrad verwendet.

Nachfolgend sind unterschiedliche Ausführungsbeispiele zur Granulierung der gemahlenen Partikel anusgeführt.

### Ausführungsbeispiel 1:

In einem offenen Mahlkreislauf hergestelltes Mahlgut in Partikelform aus 50 % Basalt, 20 % Hochofenschlacke, 30 % Quarzsand und Spuren von Tonmehl werden in einen Eirich Labormischer R02 gegeben. Um die Körnungszielwerte zu erreichen sind etwas längere Mischzeiten erforderlich. Es wird ein sehr einheitliches Körnungsband erzeugt, wobei die Bildung von zu viel Feinkorn < 1 mm vermieden werden soll.

**Folgende Parameter werden dabei verwendet:**

| Ausgangsmaterial | % | g |
|---|---|---|
| Mahlgut | 87-87,7 | 3000 |
| Wasser | 12,3-13 (14 -15 % bezogen auf Feststoff) | 420-450 |
| gesamt | 100 | 3300 |

Zusätzlich zu den angegebenen Ausgangsstoffen wurden je Mischercharge nach Abschluss der Granulierung die Formkörper mit ca. 5 % Mahlgut abgepudert, um transport- und dosierfähige Formkörper, insbesondere Grüngranulat, zu erzeugen, die weiterverarbeitet werden können.

Für einen günstigen Mischablauf zeigen sich nachfolgende Prozessschritte mit folgenden Parametern für Partikel aus dem offenen Mahlkreislauf als günstig:

| Nr. | Schritt | Masse [g] | Zeit [s] | Wirblerdrehzahl [rpm] |
|---|---|---|---|---|
| 1 | Füllen mit Feststoffpartikeln | 3000 | - | - |
| 2 | Mischen | - | 30 | 3000 |
| 3 | Füllen mit Fluid, vorzugsweise Wasser | 420-450 | - | - |
| 4 | Mischen | - | 30-60 | 3000 |
| 5 | Mischen | - | 480-600 | 1500 |
| 6 | Füllen mit Feststoffpartikeln (Pudern) | 150 | - | - |
| 7 | Mischen | - | 10 | 1500 |
| 8 | Entleeren | - | - | - |

Das bedeutet, nach der Befüllung mit Feststoffpartikeln und Fluid, insbesondere Wasser, kompaktiert das Material bei vergleichsweise kurzer Mischzeit aber hoher Drehzahl, dh hohem Energieeintrag, zu einem Mikrogranulat, welches dass bei geringer Drehzahl, dh geringem Energieeintrag, zu gröberem Granulat wächst. Dieser Wachstumsprozess ist mit der Zugabe von Fluid und Feststoff beeinflussbar. Nachteilig dabei ist, dass die Gefahr der Entstehung von größeren Unterkornanteilen besteht.

### Ausführungsbeispiel 2

Die Herstellung der Formkörper erfolgt in einem Eirich Mischer Baugröße R11. Es werden ebenfalls aus einem offenen Mahrkreislauf hergestellte Partikel entsprechend der Zusammensetzung in Ausführungsbeispiel 1 verwendet.

**Rezeptur für die Herstellung, insbesondere Granulierung am R11**

| Ausgangsmaterial | % | kg |
|---|---|---|
| Mahlgut | 87 | 200 |
| Wasser | 13 (15 % bezogen auf Feststoff) | 30 |
| gesamt | 100 | 230 |

**Mischablauf für die Granulierung am R11 für Partikel aus dem offenen Mahlkreislauf**

| Nr. | Schritt | Masse [kg] | Zeit [s] | Wirblerdrehzahl [rpm] |
|---|---|---|---|---|
| 1 | Füllen mit Feststoffpartikeln | 200 | - | - |
| 2 | Füllen mit Fluid, vorzugsweise Wasser und Mischen | 30 | 60 | 800 |
| 3 | Mischen | - | 30 | 800 |
| 4 | Mischen | - | 600 | 300 |
| 5 | Füllen mit Feststoffpartikeln (Pudern) | 10 | - | - |
| 6 | Mischen | - | 10 | 300 |
| 7 | Entleeren | - | - | - |

Die Fluidmenge kann auch auf 12 % (24 kg) bezogen auf die Feststoffpartikel reduziert werden, was günstig für die Materialfeuchte (Transportfähigkeit) des Grüngranulats und damit auch für das nachfolgende Tempern ist. Die Festigkeit des Granulates verringerte sich dabei nicht. Mit der Verringerung der Fluidmenge erhöht sich jedoch auch die benötigte Granulierzeit bei niedriger Drehzahl, um Granulat in der gewünschten Körnung zu erzeugen.

### Ausführungsbeispiel 3

Es wird versucht den Mischablauf weiter zu optimieren, wobei der Gesamtmischablauf für einen möglichen Automatikbetrieb geeignet sein soll und zugleich nicht zusätzliches Unterkorn entsteht. Das Ausgangsmaterial ist wie in Ausführungsbeispiel 1 und 2 zusammengesetzt und gemahlen.

| Ausgangsmaterial | % | kg |
|---|---|---|
| Mahlgut | 88,9 | 200 |
| Wasser | 11,1 (12,5 % bezogen auf Feststoff) | 25 |
| gesamt | 100 | 225 |

**Mischablauf für die Granulierung am R11 für Partikel aus dem offenen Mahlkreislauf**

| Nr. | Schritt | Masse [kg] | Zeit [s] | Wirblerdrehzahl [rpm] |
|---|---|---|---|---|
| 1 | Füllen mit Feststoffpartikeln | 200 | - | - |
| 2 | Füllen mit Fluid, vorzugsweise Wasser und Mischen | 25 | 60 | 800 |
| 3 | Mischen | - | 30 | 800 |
| 4 | Mischen | - | 600-720 | 300 |
| 5 | Füllen mit Feststoffpartikeln (Pudern) | 10 | - | - |
| 6 | Mischen | - | 10 | 300 |
| 7 | Entleeren | - | - | - |

### Ausführungsbeispiel 4

Die Zusammensetzung der Partikel entspricht jener der Ausführungsbeispiele 1 bis 3, wobei die Partikel in einem geschlossenen Mahlkreislauf gewonnen wurden. Das Ausgangsmaterial ist schwieriger zu granulieren.

**Rezeptur für die Granulierung am R11**

| Ausgangsmaterial | % | kg |
|---|---|---|
| Mahlgut | 88,9 | 200 |
| Wasser | 11,1 (12,5 % bezogen auf Feststoff) | 25 |
| gesamt | 100 | 225 |

**Mischablauf für die Granulierung am R11 für Partikel aus dem geschlossenen Mahlkreislauf**

| Nr. | Schritt | Masse [kg] | Zeit [s] | Wirblerdrehzahl [rpm] |
|---|---|---|---|---|
| 1 | Füllen mit Feststoffpartikeln | 200 | - | - |
| 2 | Füllen mit Fluid, vorzugsweise Wasser und Mischen | 25 | 60 | 800 |
| 3 | Mischen | - | 30 | 800 |
| 4 | Mischen | - | 1200-1500 | 300 |
| 5 | Füllen mit Feststoffpartikeln (Pudern) | 10 | - | - |
| 6 | Mischen | - | 10 | 300 |
| 7 | Entleeren | - | - | - |

Es ist eine deutlich längere Mischzeit erforderlich.

### Ausführungsbeispiel 5

Um die längere Mahlzeit der bei der Verarbeitung im geschlossenen Mahlkreis gewonnenen Partikel zu reduzieren, wird ein alternativer Mischablauf angeführt. Es wird versucht das anfangs erzeugte Mikrogranulat durch zwischenzeitliche Zugabe von Fluid und Feststoff schneller wachsen zu lassen. Im Gegensatz zu ähnlichen Versuchen zu im offenen Mahlkreislauf hergestellten Partikeln funktioniert dies in gewissen Grenzen mit Partikeln aus dem geschlossenen Mahlkreis besser. Allerdings ist der dabei entstehende Feinkornanteil augenscheinlich höher.

**Mischablauf für Granulierung am R11 für Partikel aus dem geschlossenen Mahlkreislauf**

| Nr. | Schritt | Masse [kg] | Zeit [s] | Wirblerdrehzahl [rpm] |
|---|---|---|---|---|
| 1 | Füllen mit Feststoffpartikeln | 160 | - | - |
| 2 | Füllen mit Fluid, vorzugsweise Wasser | 23 | 60 | 800 |
| 3 | Mischen | - | 10 | 800 |
| 4 | Mischen | - | 180 | 400 |
| 5 | Füllen mit Feststoffpartikeln (Pudern) | 40 | - | - |
| 6 | Füllen mit Fluid, vorzugsweise Wasser | 2 | - | 400 |
| 7 | Mischen | - | 120 | 400 |
| 8 | Mischen | - | 600-900 | 250 |
| 9 | Füllen mit Feststoffpartikeln (Pudern) | 10 | - | - |
| 6 | Mischen | - | 10 | 250 |
| 7 | Entleeren | - | - | - |

Daraus resultiert dass Partikel aus dem offenen Mahlkreis besser geeignet sind, da hier die Mischzeiten bei gleicher Rezeptur geringer sind. Für Partikel aus dem geschlossenen Kreislauf ergeben sich zwar längere Mischzeiten aber gleichzeitig weniger Feinkorn. Bei der Verwendung der Partikel aus dem offenen Mahlkreis ergeben sich kürzere Mischzeiten aber mehr Feinkorn, wobei hier ein Unterkornrecycling angebracht werden kann.

Partikel aus dem offenen Mahlkreislauf lassen sich gegenüber Partikel aus dem geschlossenen Mahlkreislauf besser, dh bei kürzerer Mischzeit mit geringem Energieeintrag, granulieren. Ursache ist wahrscheinlich die feinere Körnung der Partikel aus dem geschlossenen Mahlkreislauf.

Ein Recycling von Unterkorn ist sowohl für Partikel aus dem offenen Mahlkreis als auch für Partikel aus dem geschlossenen Mahlkreis möglich. Hierbei wird bei vergleichsweise geringem Zeitaufwand das Unterkorn abgesiebt und der Granulierung, z.B. als Ersatz des bei hohem Energieeintrag erzeugten Mikrogranulats, wieder zugeführt. Dabei kann einerseits Rohmaterial eingespart als auch klassiertes, praktisch unterkornfreies Produkt erzeugt werden.

Nach Entstehung des Mikrogranulats ist somit bei hohem Energieeintrag und bei niedrigem Energieeintrag aber hinreichend langer Mischzeit ein Granulatwachstum in den Zielkornbereich hinein möglich.

Die Produktausbeute für getemperte Formkörper beträgt für Partikel sowohl aus dem offenen als auch dem geschlossenen Mahlkreislauf zwischen 70 % und 95 %, insbesondere 87 % bis 88 %.

Die vorstehend angeführten Parameter zum Granulierprozess sind nur exemplarisch zu verstehen. Der Granulierprozess ist bezüglich der Mischzeit und Produktkörnung beeinflussbar. Die Beeinflussung ist hauptsächlich durch die zwischenzeitliche Zugabe von Fluid und Feststoff während des Mischablaufs möglich. Es besteht jedoch die Gefahr der Erzeugung größerer Mengen an Unterkorn <1 mm.

Mithilfe des erfindungsgemäßen Verfahrens können Basaltfasern, wie sie in der Patentanmeldung "Basaltfasern" der Anmelderin vom 23. 12. 2010 offenbart sind, hergestellt werden, womit auch deren Inhalt in dieser Anmeldung als offenbart gilt.

Die Ausführungsbeispiele zeigen mögliche Ausführungsvarianten des erfindungsgemäßen Verfahrens, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Die den eigenständigen erfinderischen Lösungen zugrundeliegende Aufgabe kann der Beschreibung entnommen werden.

## Patentansprüche

1. Verwendung von artifiziell hergestellten Formkörpern hergestellt gemäß einem Verfahren zur Vorbehandlung von Ausgangsmaterial zur Herstellung einer mineralischen Schmelze für die Produktion von endlosen Mineralfasern, insbesondere Basaltfasern, wobei das Ausgangsmaterial, welches Basalt und zumindest ein Bindemittel, ausgewählt aus einer Gruppe umfassend Wasser, Ton, Bentonit, Sulfitlauge, Zellulosederivate, Saccharide, Zucker, Stärke, Melasse, Zement, Phosphate, siliziumorganische Substanzen, in einer Menge mit einer unteren Grenze von 1 % und einer oberen Grenze von 40 %, Quarzsand und Schlacke, insbesondere Hochofenschlacke umfasst, zu Partikeln gemahlen wird, aus den Partikeln Formkörper hergestellt werden und die Formkörper bei einer Temperatur von ca. 300°C±50°C getempert werden.

2. Verwendung nach Anspruch 1, **dadurch gekennzeichnet, dass** das Ausgangsmaterial eine Restfeuchte mit einer unteren Grenze von 1 % und einer oberen Grenze von 20 % aufweist.

3. Verwendung nach Anspruch 1 oder 2, **dadurch gekennzeichnet, dass** das Ausgangsmaterial zu Partikeln mit einer Größe von weniger als 500 µm, insbesondere 200 µm, vorzugsweise 100 µm, gemahlen wird.

4. Verwendung nach einem der Ansprüche 1 bis 3, **dadurch gekennzeichnet, dass** die Formkörper durch Befeuchtung und gegebenenfalls Mischung, Granulierung, Pelletierung, Extrusion, Sprühtrocknung oder Expandierung hergestellt werden.

5. Verwendung nach einem der Ansprüche 1 bis 4, **dadurch gekennzeichnet, dass** die Formkörper zumindest bereichsweise mit einer Beschichtung versehen werden.

6. Verwendung nach Anspruch 5, **dadurch gekennzeichnet, dass** die Beschichtung mit Stoffen ausgewählt aus einer Gruppe umfassend Mineralmehl, Filterstaub, Flugasche, Schlackenmehl, Ton, durchgeführt wird.

7. Verwendung nach Anspruch 5 oder 6, **dadurch gekennzeichnet, dass** eine Bindung der Beschichtung durch das Bindemittel nach Anspruch 1 und/oder andere Bindemittel, wie organische Bindemittel ausgewählt aus einer Gruppe umfassend Stoffe auf Basis von Zucker, Lignin, Ligninsulfonate und/oder anorganische Bindemittel ausgewählt aus einer Gruppe umfassend Stoffe auf Basis von Zement, Kieselsäurelösungen durchgeführt wird.

8. Verwendung nach einem der Ansprüche 1 bis 7, **dadurch gekennzeichnet, dass** zumindest ein Beschleuniger ausgewählt aus einer Gruppe umfassend CO₂, Wasserglas, Gips, CaCl, zur Herstellung der Formkörper beigemengt wird.

9. Verwendung nach Anspruch 8, **dadurch gekennzeichnet, dass** eine Menge des Beschleunigers mit einer oberen Grenze von 20 % und einer unteren Grenze von 0,1 % zugesetzt wird.

10. Verwendung nach einem der Ansprüche 1 bis 9, **dadurch gekennzeichnet, dass** Formkörper, insbesondere Grüngranulat, mit einer Größe mit einer oberen Grenze von 30 mm und einer unteren Grenze von 1 mm, vorzugsweise 10 mm, hergestellt werden.

## Claims

1. Use of artificially produced molded bodies manufactured according to a process for pre-treatment of raw material to create a mineral melt for producing continuous mineral fibers, particularly basalt fibers, wherein the starting material, which comprises basalt and at least one binder selected from a group comprising of water, clay, bentonite, sulfite liquor, cellulose derivatives, saccharides, sugar, starch, molasses, cement, phosphates, organosilicon substances in a quantity having a lower limit of 1% and an upper limit of 40%, quartz sand and slag, in particular blast furnace slag, is ground into particles, molded bodies are prepared from the particles, and the molded bodies are annealed at a temperature of about 300 °C ± 50 °C.

2. Use according to claim 1, **characterized in that** the starting material has a residual moisture content with a lower limit of 1% and an upper limit of 20%.

3. Use according to claim 1 or 2, **characterized in that** the starting material is ground to form particles having a size of less than 500 µm, particularly 200 µm, preferably 100 µm.

4. Use according to any one of claims 1 to 3, **characterized in that** the molded bodies are produced by wetting and optionally mixing, granulating, pelletizing, extruding, spray drying or expansion.

5. Use according to any one of claims 1 to 4, **characterized in that** the molded bodies are at least partially provided with a coating.

6. Use according to claim 5, **characterized in that** the coating is carried out with substances selected from a group comprising mineral flour, filter dust, fly ash, slag powder, clay.

7. Use according to claim 5 or 6, **characterized, in that** binding of the coating is performed by the binder according to claim 1 and/or other binders such as organic binders selected from a group comprising substances based on sugar, lignin, lignosulfonates, and/or inorganic binding agents selected from a group comprising substances based on cement, silica solutions.

8. Use according to any one of claims 1 to 7, **characterized in that** at least one accelerant selected from a group comprising CO₂, water glass, gypsum, CaCl, is added in order to produce the molded bodies.

9. Use according to claim 8, **characterized, in that** a quantity of the accelerant with an upper limit of 20% and a lower limit of 0.1% is added.

10. Use according to any one of claims 1 to 9, **characterized in that** molded bodies, particularly green granules are prepared with a size having an upper limit of 30 mm and a lower limit of 1 mm, preferably 10 mm.

## Revendications

1. Utilisation d'objets moulés fabriqués artificiellement, fabriqués par un procédé destiné au prétraitement d'une matière de départ pour la fabrication d'une masse fondue minérale dans le but de produire des fibres minérales continues, en particulier des fibres de basalte, la matière de départ, qui comprend du basalte et au moins un liant, choisie dans un groupe comprenant l'eau, l'argile, la bentonite, la lessive sulfitique, les dérivés de la cellulose, les saccharides, les sucres, l'amidon, la mélasse, le ciment, les phosphates, les substances organosiliciées, en une quantité présentant une limite inférieure de 1 % et une limite supérieure de 40 %, le sable quartzeux et le laitier, en particulier le laitier de hauts fourneaux, étant broyée pour donner des particules, des objets moulés étant fabriqués à partir des particules, et les objets moulés étant recuits à une température d'environ 300 °C ± 50 °C.

2. Utilisation selon la revendication 1, **caractérisée en ce que** la matière de départ présente une humidité résiduelle présentant une limite inférieure de 1 % et une limite supérieure de 20 %.

3. Utilisation selon la revendication 1 ou 2, **caractérisée en ce que** la matière de départ est broyée pour donner des particules présentant une granulométrie inférieure à 500 µm, en particulier de 200 µm, de préférence de 100 µm.

4. Utilisation selon l'une des revendications 1 à 3, **caractérisée en ce que** les objets moulés sont fabriqués par humidification et éventuellement par mélange, granulation, pastillage, extrusion, séchage par atomisation ou expansion.

5. Utilisation selon l'une des revendications 1 à 4, **caractérisée en ce que** les objets moulés sont, au moins par zones, pourvus d'un revêtement.

6. Utilisation selon la revendication 5, **caractérisée en ce que** le revêtement est réalisé avec des substances choisies dans un groupe comprenant une poudre minérale, les poussières de filtration, les cendres volantes, la farine de scories, l'argile.

7. Utilisation selon la revendication 5 ou 6, **caractérisée en ce qu'**on procède à une liaison du revêtement par le liant selon la revendication 1 et/ou d'autres liants, tels que les liants organiques choisis dans un groupe comprenant les substances à base de sucres, de lignine, de ligninesulfonates et/ou les liants inorganiques choisis dans un groupe comprenant les substances à base de ciment, de solutions de silice.

8. Utilisation selon l'une des revendications 1 à 7, **caractérisée en ce qu'**on ajoute pour la fabrication des objets moulés un accélérateur choisi dans un groupe comprenant le CO₂, la solution silicatée, le gypse, le CaCl.

9. Utilisation selon la revendication 8, **caractérisée en ce qu'**on ajoute une quantité de l'accélérateur présentant une limite supérieure de 20 % et une limite inférieure de 0,1 %.

10. Utilisation selon l'une des revendications 1 à 9, **caractérisée en ce qu'**on fabrique des objets moulés, en particulier un granulé vert, dont la granulométrie présente une limite supérieure de 30 mm et une limite inférieure de 1 mm, de préférence de 10 mm.
